# EUROPEAN PATENT SPECIFICATION

(11) **EP 2 281 880 B1**
(45) Date of publication and mention of the grant of the patent: **06.07.2016**
(21) Application number: 09729276.7
(22) Date of filing: 05.03.2009
(51) Int. Cl.: C12N 15/00, C12P 13/00

(54) **MUTANT MICROORGANISM WITH HIGH ABILITY OF PRODUCING PUTRESCINE AND PREPARATION OF PUTRESCINE USING SAME**
MUTIERTER MIKROORGANISMUS MIT HOHEM PUTRESCIN-PRODUKTIONSVERMÖGEN UND HERSTELLUNG VON PUTRESCIN DAMIT
MICRO-ORGANISME MUTANT PRÉSENTANT UNE APTITUDE ÉLEVÉE À PRODUIRE DE LA PUTRESCINE ET PROCÉDÉ DE PRÉPARATION DE PUTRESCINE À L'AIDE DE CE MICRO-ORGANISME

(30) Priority: 10.04.2008 KR 20080033125
(43) Date of publication of application: 09.02.2011
(73) Proprietor: Korea Advanced Institute of Science and Technology, Daejeon 305-701 (KR)
(72) Inventor: LEE, Sang Yup, Daejeon 305-761 (KR); QIAN, Zhi Gang, Daejeon 305-701 (KR); XIA, Xiaoxia, Deajeon 305-701 (KR); JEON, Yong Jae, Daejeon 305-701 (KR)
(74) Representative: Hiebl, Inge Elisabeth
(86) International application number: PCT/KR2009/001103
(87) International publication number: WO 2009/125924

(56) References cited:
- WO-A1-2006/005603
- D. Tholl ET AL: "Retarded growth of an Escherichia coli mutant de cient in spermidine synthase can be unspeci cally repaired by addition of various polyamines", World Journal of Microbiology & Biotechnology, vol. 14, 1 January 1998 (1998-01-01), pages 857-863, XP055077913, Retrieved from the Internet: URL:http://rd.springer.com/content/pdf/10. 1023/A:1008829008065.pdf [retrieved on 2013-09-05]
- YOUNG-JOON LEE ET AL: "Genetic manipulation of a primary metabolic pathway for l-ornithine production in Escherichia coli", BIOTECHNOLOGY LETTERS, SPRINGER NETHERLANDS, DORDRECHT, vol. 28, no. 22, 25 August 2006 (2006-08-25), pages 1849-1856, XP019433302, ISSN: 1573-6776, DOI: 10.1007/S10529-006-9163-Y
- KASHIWAGI K ET AL: "Coexistence of the genes for putrescine transport protein and ornithine decarboxylase at 16 min on Escherichia coli chromosome", JOURNAL OF BIOLOGICAL CHEMISTRY, AMERICAN SOCIETY FOR BIOCHEMISTRY AND MOLECULAR BIOLOGY, vol. 266, no. 31, 5 November 1991 (1991-11-05), pages 20922-20927, XP002318344, ISSN: 0021-9258
- S. KURIHARA ET AL: "The Putrescine Importer PuuP of Escherichia coli K-12", JOURNAL OF BACTERIOLOGY, vol. 191, no. 8, 30 January 2009 (2009-01-30), pages 2776-2782, XP055077923, ISSN: 0021-9193, DOI: 10.1128/JB.01314-08
- S. KURIHARA ET AL: "-Glutamylputrescine Synthetase in the Putrescine Utilization Pathway of Escherichia coli K-12", JOURNAL OF BIOLOGICAL CHEMISTRY, vol. 283, no. 29, 30 April 2008 (2008-04-30), pages 19981-19990, XP055070572, ISSN: 0021-9258, DOI: 10.1074/jbc.M800133200
- JENS SCHNEIDER ET AL: "Biotechnological production of polyamines by Bacteria: recent achievements and future perspectives", APPLIED MICROBIOLOGY AND BIOTECHNOLOGY, SPRINGER, BERLIN, DE, vol. 91, no. 1, 7 May 2011 (2011-05-07), pages 17-30, XP019915706, ISSN: 1432-0614, DOI: 10.1007/S00253-011-3252-0
- BANDOUNAS L ET AL: "Putrescine production from glucose by Pseudomonas putida S12-Identification of genes involved in putrescine degradation using a transcriptomics approach", JOURNAL OF BIOTECHNOLOGY, ELSEVIER SCIENCE PUBLISHERS, AMSTERDAM, NL, vol. 131, no. 2, 1 September 2007 (2007-09-01), page S207, XP027494730, ISSN: 0168-1656, DOI: 10.1016/J.JBIOTEC.2007.07.371 [retrieved on 2007-09-01]
- ZHI-GANG QIAN ET AL: "Metabolic engineering of Escherichia coli for the production of putrescine, a four carbon diamine", BIOTECHNOLOGY AND BIOENGINEERING, vol. 104, no. 4, 1 November 2009 (2009-11-01), pages 651-662, XP055032553, ISSN: 0006-3592, DOI: 10.1002/bit.22502
- FUKUCHI, J.I. ET AL.: 'Decrease in cell viability due to the accumulation of spermidine in spermidine acetyltransferase-deficient mutant of Escherichia coli' BIOLOGICAL CHEMISTRY vol. 270, no. 32, 1995, pages 18831 - 18835, XP002300277
- PANAGIOTIDIS, C.A. ET AL.: 'Biosynthesis ofpolyamines in ornithine decarboxylase, arginine decarboxylase, and agmatine ureohydrolase deletion mutants of Escherichia coli strain K-12' PROC. NATL. ACAD. SCI. USA vol. 84, 1987, pages 4423 - 4427, XP002928931
- OSHIMA, T.: 'Unique polyamines produced by an extreme thermophilie, Thermus thermophilus' AMINO ACIDS vol. 33, 2007, pages 367 - 372, XP019544760
- VLIET, F.V. ET AL.: 'Evolutionary divergence of genes for ornithine and aspartate carbamoyl-transferases- complete sequence and mode of regulation of the Escherichia coli argF gene; comparison of argF with arg1 and pyrB' NUCLEIC ACID RESEARCH vol. 12, no. 15, 1984, pages 6277 - 6289, XP002975548
- CHATTOPADHYAY, M.K. ET AL.: 'Methylthioadenosine and poluamine biosynthesis in a Sacchromyces cerevisiae meul mutant' BIOCHEMICAL AND BIOPHYSICAL RESEARCH COMMUNICATIONS vol. 343, 2006, pages 203 - 207, XP008136777
- BOYLE, S.M. ET AL.: 'Expression of the cloned genes encoding the putrescine biosynthetic enzymes and methionine adenosyltransferase of Escherichia coli(speA, speB, speC and metK)' GENE vol. 30, 1984, pages 129 - 136, XP025696522
- KURIHARA, S. ET AL.: 'A novel putrescine utilization pathway involves r-glutamylated intermediates of Escherichia coli K-12' J. BIOLIGICAL CHEMISTRY vol. 280, 2005, pages 4602 - 4608, XP008136778
- SAMUEL RAJ, V. ET AL.: 'Properties of a revertant of Escherichia coli viable in the presence of spermidine accumulation: Increase in L-glycerol3-phosphate' J. BACTERIOLOGY vol. 183, 2001, pages 4493 - 4498, XP008136779
- MATSUDA I. ET AL.: 'Molecular basis of urea cycle disorders' NIPPON RINSHO. vol. 51, 1993, pages 520 - 524, XP008136789
- SAVCHENKO A ET AL: "The Bacillus stearothermophilus argCJBD operon harbours a strong promoter as evaluated in Escherichia coli cells", GENE, ELSEVIER, AMSTERDAM, NL, vol. 212, no. 2, 8 June 1998 (1998-06-08), pages 167-177, XP004122918, ISSN: 0378-1119, DOI: 10.1016/S0378-1119(98)00174-7

## Description

### TECHNICAL FIELD

The present invention relates to mutant microorganisms having high ability to produce putrescine and a method for producing putrescine using the same, and more particularly to mutant microorganisms having high ability to produce putrescine in which genes involved in the putrescine degradation or utilization pathway are inactivated or deleted, and to a method of producing putrescine in high yield by culturing the mutant microorganisms.

### BACKGROUND ART

Putrescine (also known as 1,4-butanediamine), an important raw material for the production of polyamide-4,6, including nylon-4,6, is mainly produced on industrial scale by the hydrogenation of acrylonitrile, which is produced by addition of hydrogen cyanide to acrylonitrile. Known processes for the chemical synthesis of this compound require non-renewable petrochemical products as raw materials, and relatively severe reaction conditions of temperature and pressure in a multi-step and multi-reactor design, as well as the use of expensive catalyst systems. Furthermore, because these raw materials are highly toxic and flammable, the known chemical synthetic processes are disadvantageous in environmental terms. Accordingly, as an alternative to the chemical production process, a process of producing putrescine from a renewable biomass-derived carbon source is required.

Putrescine is a kind of polyamine which is found in a variety of organisms ranging from bacteria to animals and plants. For example, putrescine is known to play an important role not only in cell proliferation and normal cell growth, but also in a defensive mechanism against oxidative stress (Tkachenko et al., Arch. Microbiol., 176:155-157, 2001). Meanwhile, the intracellular contents of polyamines are tightly controlled by their biosynthesis, degradation, degradation, uptake and excretion (Igarashi and Kashiwagi et al., J. Bacteriol., 170(7):3131-3135, 1988). As is known in the art, the concentration of putrescine in *E. coli* is as extremely high as about 2.8 g/L. Also, microorganisms have potentially good resistance to high concentrations of polyamines. For example, Mimitsuka et al. reported that *Corynebacterium glutamicum* can grow even in the presence of more than 30 g/L of cadaverine. Accordingly, studies on the use of microorganisms for producing high concentrations of polyamines (putrescine) have been continued.

EP 0726240 A1 discloses a method of producing putrescine through fermentation using either inexpensive industrial waste products or materials having protein as a major constituent. However, because the disclosed materials are very complex, there is a problem in that many purification steps have to be carried out in order to obtain putrescine and cadaverine products. In addition, European Patent Publication No. 1784496 A1 discloses a process of biochemically synthesizing putrescine by microbial growth in minimal salt medium containing glucose as a carbon source. In this European patent publication, in order to improve the conversion of ornithine to putrescine, the activity of ornithine decarboxylase was increased by overexpression of an ornithine decarboxylase-encoding *speC* or *speF.* However, when the putrescine content is increased as a result of increasing ornithine decarboxylase, there are problems in that putrescine biosynthesis is reduced and the degradation of putrescine is induced (Igarashi and Kashiwagi et al., Biochem. J., 347:297-303, 2000).

Studies on the degradation and utilization of putrescine in microorganisms are as follows. Bowman et al. reported that spermidine synthase which is the product of the *speE* gene promotes the biosynthesis of spermidine from putrescine in *E*. *coli* (Bowman et al., J. Biol. Chem., 248:2480-2486, 1973). spermidine synthase (EC:2.5.1.16) is present in most cell systems for the synthesis of spermidine.

Haywood et al. reported that the yeast *Candida boidinii* acetylates putrescine to N-acetylputrescine by N-acetyltransferase. Spermidine acetyltransferase which is the *E. coli speG* gene product has high homology with the N-acetyltransferase of the yeast, and thus must possess putrescine acetyltransferase (Haywood and Large, Eur J. Biochem., 148:277-283, 1985).

Furthermore, Samsonova et al. reported another putrescine degradation pathway in which a coupling action of *E. coli* YgjG putrescine transaminase and YdcW dehydrogenase resulted in conversion of putrescine into γ-aminobutyric acid without γ-glutamylation (Samsonova et al., BMC Microbiol., 3:2, 2003; Samsonova et al., FEBS Lett., 579:4107-4112, 2005).

Moreover, Kurihara et al. reported that the putrescine degradation pathway, the 'Puu pathway', involves γ-glutamylated intermediates of *E. coli.* Through γ-glutamylation of putrescine in this pathway, γ-aminobutyraldehyde which is an aldehyde intermediate can be stabilized. γ-glutamylputrescine synthetase which is the product of the *puuA* gene converts putrescine to γ-glutamyl-L-putrescine while promoting the first reaction of this pathway. Also, it was revealed that the catabolic pathway is the main one when *E. coli* grows in a medium containing putrescine as the sole source of nitrogen. In addition, it was revealed that the putrescine importer *puuP* is involved in the catabolic pathway and is the main putrescine importer when *E. coli* grows in a medium containing putrescine as a sole nitrogen source (Kurihara et al., J. Biol. Chem., 280:4602-4608, 2005).

Accordingly, the present inventors have prepared mutant microorganisms in which at least one gene selected from a gene (*speE*) encoding spermidine synthase, a gene (*speG*) encoding spermidine N-acetyltransferase, a gene (*argI*) encoding ornithine carbamoyltransferase chain I-monomer and a gene (*puuP*) encoding putrescine importer, which are involved in the putrescine degradation or utilization pathway of putrescine-producing microorganisms, is inactivated or deleted. Also, the preset inventors have found that, when the mutant microorganisms are cultured, they can produce putrescine in high yield, thereby completing the present invention.

### SUMMARY OF INVENTION

It is an object of the present invention to provide mutant microorganisms in which at least one gene involved in the putrescine degradation or utilization pathway is inactivated or deleted and which have high ability to produce putrescine, and a method for preparing the mutant microorganisms.

Another object of the present invention is to provide a method of producing putrescine in high yield by culturing the mutant microorganisms.

To achieve the above objects, the present invention provides a mutant microorganism having the ability to produce putrescine, wherein at least one gene selected from the group consisting of a speE gene encoding spermidine synthase, a speG gene encoding spermidine N-acetyltransferase, an argI gene encoding ornithine carbamoyltransferase chain I-monomer and a puuP gene encoding putrescine importer, which are involved in the putrescine degradation or utilization pathway, is inactivated or deleted; and wherein at least one gene selected from the group consisting of a puuA gene encoding γ-glutamylputrescine synthase, a ygjG gene encoding putrescine transaminase and an argF gene encoding ornithine carbamoyltransferase F is further inactivated or deleted; and wherein a promoter of at least one gene selected from the group consisting of an argECBH gene encoding an operon for arginine biosynthesis, an argD gene encoding acetylornithine aminotransferase, and a speF-potE gene encoding inducible ornithine decarboxylase and putrescine/ornithine antiporter is replaced with a promoter selected from the group consisting of a trc promoter, a tac promoter, a T7 promoter, a lac promoter and a trp promoter; and selected from the group consisting of *Bacillus sp.* and *Escherichia sp..*
The present invention also provides a mutant microorganism as described directly above, wherein a speC gene encoding ornithine decarboxylase is further introduced or amplified.

The present invention also provides a method of preparing a mutant microorganism selected from the group consisting of *Bacillus sp.* and *Escherichia sp.* having the ability to produce putrescine, the method comprising: in-activating or deleting at least one gene selected from the group consisting of a speE gene encoding spermidine synthase, a speG gene encoding spermidine N-acetyltransferase, an argI gene encoding ornithine carbamoyltransferase I and a puuP gene encoding putrescine importer, which are involved in the putrescine degradation or utilization pathway, from a micro-organism having a putrescine production pathway; and at least one gene selected from the group consisting of a puuA gene encoding γ-glutamylputrescine synthase, a ygjG gene encoding putrescine transaminase and an argF gene encoding ornithine carbamoyltransferase F is further inactivated or deleted.
The present invention also provides a method for producing putrescine, the method comprising culturing said mutant microorganism to produce putrescine, and then collecting putrescine from the culture broth.

Other features and aspects of the present invention will be more apparent from the following detailed description and the appended claims.

### BRIEF DESCRIPTION OF DRAWINGS

FIG. 1 is a schematic diagram showing a pathway for the synthesis of putrescine from glucose.
FIG. 2 is a graphic diagram showing the production of putrescine from XQ37/pKKSpeC cells in fed-batch fermentation using glucose.
FIG. 3 is a graphic diagram showing the production of putrescine from XQ39 cells in fed-batch fermentation using glucose.
FIG. 4 is a graphic diagram showing the production of putrescine from XQ43 cells in fed-batch fermentation using glucose.
FIG. 5 is a graphic diagram showing the production of putrescine from XQ43/p15SpeC cells in fed-batch fermentation using glucose.

### DETAILED DESCRIPTION OF THE INVENTION, AND PREFERRED EMBODIMENTS

As used herein, the term "inactivated" is meant to include mutating, replacing or deleting part of the gene of interest, or introducing one or more bases into the gene, so as to reduce the activity of an enzyme, which is expressed by the gene, thereby blocking part, or a substantial part, of the biosynthetic pathway in which the enzyme of the gene is involved.

As used herein, the term "deleted" is meant to include mutating, replacing or deleting part or all of the gene of interest, or introducing one or more bases into the gene, such that the gene is not expressed or does not exhibit enzymatic activity, even though it is expressed, thereby blocking the biosynthetic pathway in which the gene is involved.

As used herein, the term "amplified" is meant to include mutating, replacing or deleting part of the gene of interest, or introducing one or more bases into the gene, or introducing a gene of another microbial origin encoding the same enzyme, so as to increase the activity of the corresponding enzyme.

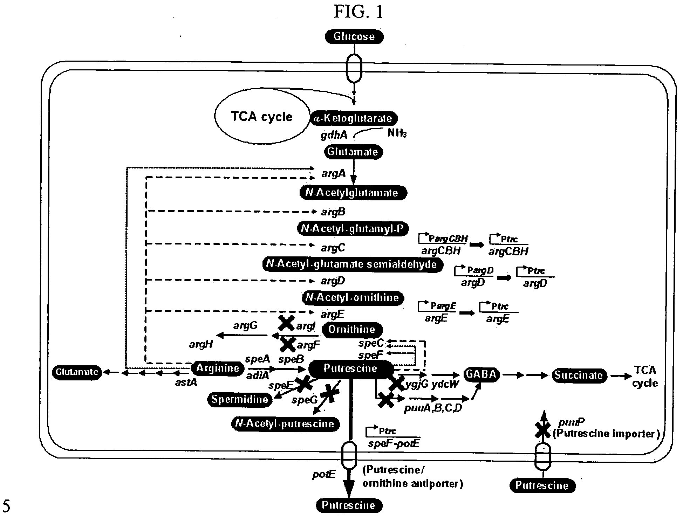

FIG. 1 is a schematic diagram showing a pathway for the synthesis of putrescine from glucose. As shown in FIG. 1, in the present invention, it was found that, when genes (*speE, speG, argI,* and *puuP*) involved in the putrescine degradation or utilization pathway of a microorganism having the ability to produce putrescine were inactivated or deleted, the mutant microorganism could produce putrescine in high yield. Reduced activities of the genes (*speE, speG, argI,* and *puuP*) involved in the putrescine degradation or utilization pathway could be confirmed by reduced transcriptional and translational efficiency as compared to those of the respective wild-type genes.

In Examples of the present invention, the present inventors prepared mutant microorganisms in which at least one selected from the group consisting of a gene (*speE*) encoding spermidine synthase, a gene (*speG*) encoding spermidine N-acetyltransferase, a gene (*argI*) encoding ornithine carbamoyltransferase chain I-monomer and a gene (*puuP*) encoding putrescine importer, which are involved in the putrescine degradation or utilization pathway, was inactivated or deleted. It was found that the prepared mutant microorganisms had an improved ability to produce putrescine.

Accordingly, in one aspect, the present invention relates to a mutant microorganism having the ability to produce putrescine in which at least one gene selected from the group consisting of a speE gene encoding spermidine synthase, a speG gene encoding spermidine N-acetyltransferase, an argI gene encoding ornithine carbamoyltransferase chain I-monomer and a puuP gene encoding putrescine importer, which are involved in the putrescine degradation or utilization pathway, is inactivated or deleted; and wherein at least one gene selected from the group consisting of a puuA gene encoding γ-glutamylputrescine synthase, a ygjG gene encoding putrescine transaminase and an argF gene encoding ornithine carbamoyltransferase F is fur-ther inactivated or deleted; and wherein a promoter of at least one gene selected from the group consisting of an ar-gECBH gene encoding an operon for arginine biosynthesis, an argD gene encoding acetylornithine aminotransferase, and a speF-potE gene encoding inducible ornithine decarboxylase and putrescine/ornithine antiporter is replaced with a promoter selected from the group consisting of a trc promoter, a tac promoter, a T7 promoter, a lac promoter and a trp promoter; and selected from the group consisting of *Bacillus sp.* and *Escherichia sp..*

Further disclosed is a mutant microorganism having the ability to produce putrescine in which at least one gene selected from the group consisting of a gene (*speE*) encoding spermidine synthase, a gene (*speG*) encoding spermidine N-acetyltransferase, a gene (*argI*) encoding ornithine carbamoyltransferase chain I-monomer and a gene (*puuP*) encoding putrescine importer, which are involved in the putrescine degradation or utilization pathway, is inactivated or deleted, and a method for producing the mutant microorganism.

In the mutant microorganism of the present invention, at least one selected from the group consisting of a gene (*puuA*) encoding γ-glutamylputrescine synthase, a gene (*ygjG*) encoding putrescine transaminase and a gene (*argF*) encoding ornithine carbamoyltransferase chain F-monomer is inactivated or deleted.

The gene (*argF*) encoding ornithine carbamoyltransferase chain F-monomer is an isoenzyme of the gene (*argI*) encoding ornithine carbamoyltransferase chain I-monomer, and the gene (*puuA*) encoding γ-glutamylputrescine synthase is a neighboring gene of the gene (*puuP*) encoding putrescine importer. Also, the gene (*ygjG*) encoding putrescine transaminase is a gene which is involved in putrescine degradation.

In the mutant microorganism of the present invention, a gene (*lacI*) encoding a lac operon repressor may also additionally be deleted such that the expression of the genes encoding the enzymes which are involved in putrescine biosynthesis is increased. Examples of the genes encoding the enzymes which are involved in putrescine biosynthesis include *gdhA, argA, argB, argC, argD, argE,* etc.

In the mutant microorganism of the present invention, a gene *(speC)* encoding ornithine decarboxylase may also additionally be introduced or amplified. The gene *(speC)* encoding ornithine decarboxylase is introduced in the form of an expression vector containing a strong promoter. The strong promoter may be selected from the group consisting of a trc promoter, a tac promoter, a T7 promoter, a lac promoter and a trp promoter.

As the microorganism in the present invention, *Bacillus sp.* or *Escherichia sp.* may be used.

In the present invention, it was found that, in the case of the mutant microorganism in which the gene involved in the putrescine degradation or utilization pathway has been deleted, when the promoter of a gene (*argECBH*) selected from the group consisting of a gene encoding an operon for arginine biosynthesis, a gene (*argD*) encoding acetylornithine aminotransferase, and a gene (*speF-potE*) encoding inducible ornithine decarboxylase and putrescine/ornithine antiporter was replaced with a strong promoter, the resulting mutant microorganism could produce putrescine in higher yield.

In Examples of the present invention, based on the mutant microorganism in which the genes (*speE, speG, argI,* and *puuP*) involved in the putrescine degradation or utilization pathway and the gene (*lacI*) encoding the lac operon repressor have been deleted, the following microorganisms were prepared: a microorganism (XQ33) in which the promoter of a gene (*argECBH*) encoding an operon for arginine biosynthesis was replaced with a strong promoter (trc); a microorganism (XQ37) in which the promoters of the gene *argECBH* and a gene (*speF-potE*) encoding inducible ornithine decarboxylase and putrescine/ornithine antiporter were replaced with the strong promoter trc; a mutant microorganism (XQ39) in which the gene *argECBH,* the gene *speF-potE* and the gene (*argD*) encoding acetylornithine aminotransferase were replaced with the strong promoter trc; and a mutant microorganism (XQ43) in which the promoters of the gene *argECBH,* the gene *speF-potE,* the gene *argD* and the gene *(speC)* encoding ornithine decarboxylase were replaced with the strong promoter (trc). It was found that the abilities of these microorganisms to produce putrescine were abruptly increased.

Accordingly, the present invention relates to a mutant microorganism having the ability to produce putrescine in which at least one gene selected from the group consisting of a speE gene encoding spermidine synthase, a speG gene encoding spermidine N-acetyltransferase, an argI gene encoding ornithine carbamoyltransferase chain I-monomer and a puuP gene encoding putrescine importer, which are involved in the putrescine degradation or utilization pathway, is inactivated or deleted; and wherein at least one gene selected from the group consisting of a puuA gene encoding γ-glutamylputrescine synthase, a ygjG gene encoding putrescine transaminase and an argF gene encoding ornithine carbamoyltransferase F is further inactivated or deleted; and wherein a promoter of at least one gene selected from the group consisting of an ar-gECBH gene encoding an operon for arginine biosynthesis, an argD gene encoding acetylornithine aminotransferase, and a speF-potE gene encoding inducible ornithine decarboxylase and putrescine/ornithine antiporter is replaced with a promoter selected from the group consisting of a trc promoter, a tac promoter, a T7 promoter, a lac promoter and a trp promoter; and selected from the group consisting of *Bacillus sp.* and *Escherichia sp.,* and a method for producing the mutant microorganism.

In the present invention, the gene (*argECBH*) encoding the operon for arginine biosynthesis is a divergent operon flanked by two convergent promoters (argEp and argCBHp) and containing an operator. The two promoters are repressed by arginine (Charlier and Glansdorff, 2004). Thus, when the native promoter of the argECBH operon is replaced with the strong promoter, the metabolic flux to ornithine can be increased. The gene *argE* is a gene encoding N-acetylornithinase, the gene *argC* is a gene encoding N-acetylglutamylphosphate reductase, the gene *argB* is a gene encoding N-acetylglutamate kinase, and the gene *argH* is a gene encoding argininosuccinase.

The gene (*speF-potE*) encoding inducible ornithine decarboxylase and putrescine/ornithine antiporter, which is induced at low pH, encodes inducible ornithine decarboxylase and putrescine/ornithine antiporter. Thus, when the native promoter of the speF-potE operon is replaced with the strong promoter, the speF-potE operon can be constitutively expressed, and thus the ability to produce putrescine can be improved.

The promoter of the gene (*argD*) encoding acetylornithine aminotransferase is repressed by arginine (Charlier and Glansdorff, 2004). Thus, when the native promoter of the argD operon is replaced with the strong promoter, the metabolic flux to ornithine can be increased.

As described above, in the mutant microorganism of the present invention, at least one selected from the group consisting of the gene (*puuA*) encoding γ-glutamylputrescine synthase, the gene (*ygjG*) encoding putrescine transaminase and the gene (*argF*) encoding ornithine carbamoyltransferase chain F-monomer is inactivated or deleted.

In the mutant microorganism having the ability to produce putrescine, the gene (/*acI*) encoding the lac operon repressor may additionally be deleted.

In the present invention, the gene *(speC)* encoding ornithine decarboxylase is preferably introduced in the form of an expression vector containing a strong promoter.

In the present invention, the strong promoter which is used in the replacement of the gene promoter and the introduction of the gene (*speC*) encoding ornithine decarboxylase is preferably selected from the group consisting of a trc promoter, a tac promoter, a T7 promoter, a lac promoter and a trp promoter.

The most preferred example of the mutant microorganism according to the present invention may be a mutant microorganism having the ability to produce putrescine in which at least one selected from the group consisting of a gene (*speE*) encoding spermidine synthase, a gene (*speG*) encoding spermidine N-acetyltransferase, a gene (*argI*) encoding ornithine carbamoyltransferase chain I-monomer and a gene (*puuP*) encoding putrescine importer, which are involved in the putrescine degradation or utilization pathway, is inactivated or deleted, and in which the promoter of at least one selected from the group consisting of a gene (*argECBH*) encoding an operon for arginine biosynthesis, a gene (*argD*) encoding acetylornithine aminotransferase and a gene (*speF-potE*) encoding inducible ornithine decarboxylase and putrescine/ornithine antiporter is replaced with a strong promoter, and in which a gene *(speC)* encoding ornithine decarboxylase is introduced or amplified, wherein said microorganism is selected from the group consisting of *Bacillus sp.* and *Escherichia sp..*

In still another aspect, the present invention relates to a method for producing putrescine, the method comprising culturing said mutant microorganism to produce putrescine, and then collecting putrescine from the culture broth.

In the present invention, the processes of culturing the mutant microorganism and collecting putrescine from the culture broth can be carried out using a conventional culture method (batch culture or fed-batch culture, known in the prior fermentation processes, and a method for the isolation and purification of putrescine, known in the art.

In the present invention, the biosynthetic production of putrescine can be carried out *in vivo* or *in vitro.*

### Examples

Hereinafter, the present invention will be described in further detail with reference to examples. It is to be understood, however, that these examples are for illustrative purposes only and are not to be construed to limit the scope of the present invention.

Particularly, although only specific kinds of vectors for removing the genes according to the present invention, and the putrescine-producing microorganisms of *Escherichia* sp. serving as host cells were illustrated in the following examples, it will also be obvious to a person skilled in the art to use other types of vectors and putrescine-producing microorganisms.

### Example 1: Preparation of mutant microorganism in which genes involved in the putrescine degradation or utilization pathway are deleted

In the present invention, the deletion of genes (*puuA, puuP, ygjG, speE, speG, argF,* and *argI*) on the chromosomes was performed by double-crossover homologous recombination (Datsenko, K.A., & Wanner, B.L. Proc. Natl. Acad. Sci., 97:6640-6645, 2000). A lox71-chloramphenicol marker (CmR)-lox66 cassette was prepared by PCR using primers containing 50 nucleotides homologous to the upstream and downstream regions of the target gene. pECmulox (Kim, J.M., Lee, K.H. & Lee, S.Y., FEMS Microbiol. Lett., 278: 78-85, 2008) comprising the lox71-CmR-lox66 cassette was used as a template in PCR reactions. The PCR products were transformed into electrocompetent cells containing λ recombinase. Colonies were selected on Luria-Bertani (LB) agar plates containing 34 µg/ml of chloramphenicol (Cm) (Sambrook, J., Fritsch E.F., & Maniatis, T., Molecular cloning: a laboratory manual, 3rd edition, Cold Spring Harbor Laboratory Press, 2000). Successful gene replacement with Cm^{R} was confirmed by direct colony PCR. The antibiotic marker was eliminated by a helper plasmid pJW168 having a temperature-sensitive replication origin and expressing the IPTG-inducible cre recombinase (Palmeros et al., Gene, 247(1):255-264, 2000).

### 1-1: Preparation of WL3110 strain

PCR was performed using plasmid pECmulox as a template and primers of SEQ ID NOS: 1 and 2, thus obtaining a PCR product in which the *lacI* gene has been deleted. Then, the obtained PCR product was electroporated into electrocompetent *E. coli* (W3110) containing λ recombinase, thus preparing a strain WL3110 (W3110 *ΔlacI*).

### 1-2: Preparation of XQ08 strain

PCR was performed using plasmid pECmulox as a template and primers of SEQ ID NOS: 3 and 4, thus obtaining a PCR product in which the *speE* gene has been deleted. Then, the obtained PCR product was electroporated into the WL3110 strain prepared in Example 1-1, thus preparing a strain XQ08 (W3110 Δ*lacI* Δ*speE*).

### 1-3: Preparation of XQ17 strain

PCR was performed using plasmid pECmulox as a template and primers of SEQ ID NOS: 5 and 6, thus obtaining a PCR product in which the *puuA* gene has been deleted. Then, the obtained PCR product was electroporated into the WL3110 strain prepared in Example 1-1, thus preparing a strain XQ17 (W3110 Δ*lacI* Δ*putcA*).

### 1-4: Preparation of XQ22 strain

PCR was performed using plasmid pECmulox as a template and primers of SEQ ID NOS: 6 and 7, thus obtaining a PCR product in which the *puuP* gene has been deleted. Then, the obtained PCR product was electroporated into the XQ17 strain (W3110 Δ*lacI* Δ*puuA*) prepared in Example 1-3, thus preparing a strain XQ22 (W3110 Δ*lacI* Δ*puuP* Δ*puuA*).

### 1-5: Preparation of XQ23 strain

PCR was performed using plasmid pECmulox as a template and primers of SEQ ID NOS: 8 and 9, thus obtaining a PCR product in which the *speE* gene has been deleted. Then, the obtained PCR product was electroporated into the WL3110 strain prepared in Example 1-1, thus preparing a strain XQ23-1 (W3110 Δ*lacI* Δ*speE*).

PCR was performed using plasmid pECmulox as a template and primers of SEQ ID NOS: 10 and 11, thus obtaining a PCR product in which the *speG* gene has been deleted. Then, the obtained PCR product was electroporated into the XQ23-1 strain (W3110 Δ*lacI* Δ*speE*), thus preparing a strain XQ23-2 (W3110 Δ*lacI* Δ*speE* Δ*speG*).

PCR was performed using plasmid pECmulox as a template and primers of SEQ ID NOS: 12 and 13, thus obtaining a PCR product in which the *argI* gene has been deleted. Then, PCR was performed using the obtained PCR product as a template and primers of SEQ ID NOS: 14 and 15, thus obtaining a final PCR product. The obtained final PCR product was electroporated into the XQ23-2 strain (W3110 Δ*lacI* Δ*speE* Δ*speG*), thus preparing a strain XQ23 (W3110 Δ*lacI* Δ*speE* Δ*speG* Δ*argI*).

### 1-6: Preparation of XQ26 strain

The PCR product prepared in Example 1-3, in which the *puuA* gene has been deleted and the PCR product prepared in Example 1-4, in which the *puuP* gene has been deleted was electroporated into the XQ23 strain (W3110 Δ*lacI* Δ*speE* Δ*speG* Δ*argI*), thus preparing a strain XQ26 (W3110 Δ*lacI* Δ*speE* Δ*speG* Δ*argI* Δ*puuP* Δ*puuA*).

### 1-7: Preparation of XQ27 strain

PCR was performed using plasmid pECmulox as a template and primers of SEQ ID NOS: 16 and 17, thus obtaining a PCR product in which the *ygiG* gene has been deleted. Then, the obtained PCR product was electroporated into XQ23-2 strain (W3110 Δ*lacI* Δ*speE* Δ*speG*) prepared in Example 1-5, thus preparing a strain XQ27-1 (W3110 Δ*lacI* Δ*speE* Δ*speG* Δ*ygiG*). Then, The PCR product prepared in Example 1-3, in which the *puuA* gene has been deleted and the PCR product prepared in Example 1-4, in which the *puuP* gene has been deleted was electroporated into the XQ27-1 strain (W3110 Δ*lacI* Δ*speE* Δ*speG* Δ*ygiG*), thus preparing a strain XQ27 (W3110 Δ*lacI* Δ*speE* Δ*speG* Δ*ygiG* Δ*puuP* Δ*puuA*).

### 1-8: Preparation of XQ29 strain

The PCR product prepared in Example 1-7, in which the *ygiG* gene has been deleted was electroporated into the XQ26 strain (W3110 Δ*lacI* Δ*speE* Δ*speG* Δ*argI* Δ*puuP* Δ*puuA*) prepared in Example 1-6, thus preparing a strain XQ29 (W3110 Δ*bacI* Δ*speE* Δ*speG* Δ*ygiG* Δ*argI* Δ*puuP* Δ*puuA*).

PCR was performed using plasmid pECmulox as a template and primers of SEQ ID NOS: 1 and 2, thus obtaining a PCR product in which the *lacI* gene has been deleted. Then, the obtained PCR product was electroporated into electrocompetent *E. coli* (W3110) containing λ recombinase, thus preparing a strain WL3110 (W3110 Δ*lacI*).

### Example 2: Replacement of promoter

In order to improve the ability to produce putrescine, the promoter of the mutant strain (XQ26) prepared in Example 1 was replaced with a strong promoter (trc).

### 2-1: Preparation of XQ33 strain

Replacement of the native promoter of the argECBH operon with the trc promoter was carried out in the following manner. A DNA fragment of fused lox71-chloramphenicol marker antibiotic marker-lox66 was generated by the first PCR reaction with primers of SEQ ID NOS: 18 and 19 using pECmulox as a template. In order to introduce the trc promoter, the second PCR reaction was performed with primers of SEQ ID NOS: 20 and 21 using the first PCR product as a template. In order to introduce homologous regions into the final PCR product, the third PCR reaction was performed with primers of SEQ ID NOS: 22 and 23 using the second PCR product as a template.

The final PCR product was electroporated into the XQ26 strain (W3110 Δ*lacI ΔspeE ΔspeG ΔargI ΔpuuP ΔpuuA*) prepared in Example 1-1, thus constructing a recombinant microorganism. Then, the cells of the mutant microorganism were cultured on an agar plate containing chloramphenicol, while cells in which double homologous recombination occurred were selected on the agar plate, thus preparing an XQ33 strain (*W3110 ΔlacI ΔspeE ΔspeG ΔargI ΔpuuP ΔpuuA PargECBH::Ptrc*). Then, replacement of the prepared strain with the trc promoter was confirmed by DNA sequence analysis.

### 2-2: Preparation of XQ37 strain

Replacement of the native promoter of the speF-potE operon with the trc promoter was performed in the following manner. The first PCR reaction was carried out with primers of SEQ ID NOS: 19 and 24 using plasmid pECmulox as a template.

The second PCR reaction was carried out with primers of SEQ ID NOS: 25 and 26 using the first PCR product as a template. The third PCR reaction was carried out with primers of SEQ ID NOS: 27 and 28 using the second PCR product as a template.

The final PCR product was electroporated into the XQ33 strain (W3110 *ΔlacI ΔspeE ΔspeG ΔargI ΔpuuP ΔpuuA PargECBH::Ptrc*) prepared in Example 2-1, thus constructing a recombinant microorganism. Then, the cells of the recombinant microorganism were cultured on an agar plate containing chloramphenicol, while cells in which double homologous recombination occurred were selected on the agar plate, thus preparing an XQ37 strain (W3110 *ΔlacI ΔspeE ΔspeG ΔargI ΔpuuP ΔpuuA PargECBH::Ptrc PspeF-potE::Ptrc*). Replacement with the trc promoter was confirmed by DNA sequence analysis.

### 2-3: Preparation of XQ39 strain

Replacement of the native promoter of the argD operon with the trc promoter was performed in the following manner. The first PCR reaction was carried out with primers of SEQ ID NOS: 19 and 29 using plasmid pECmulox as a template.

The second PCR reaction was carried out with primers of SEQ ID NOS: 30 and 31 using the first PCR product as a template. The third PCR reaction was carried out with primers of SEQ ID NOS: 32 and 33 using the second PCR product as a template.

The final PCR product was electroporated into the XQ37 strain (W3110 *ΔlacI ΔspeE ΔspeG ΔargI ΔpuuP ΔpuuA PargECBH::Ptrc PspeF-potE::Ptrc*) prepared in Example 2-2, thus preparing an XQ39 strain (W3110 *ΔlacI ΔspeE ΔspeG ΔargI ΔpuuP ΔpuuA PargECBH::Ptrc PspeF-potE::Ptrc PargD::Ptrc*). Then, the cells of the recombinant microorganism were cultured on an agar plate containing chloramphenicol, while cells in which double homologous recombination occurred were selected on the agar plate. Replacement with the trc promoter was confirmed by DNA sequence analysis.

### 2-4: Preparation of XQ43 strain

Replacement of the native promoter of the *speC* gene with the trc promoter was performed in the following manner. The first PCR reaction was carried out with primers of SEQ ID NOS: 19 and 36 using plasmid pECmulox as a template.

The second PCR reaction was carried out with primers of SEQ ID NOS: 37 and 38 using the first PCR product as a template. The third PCR reaction was carried out with primers of SEQ ID NOS: 39 and 40 using the second PCR product as a template.

The final PCR product was electroporated into the XQ39 strain (W3110 *ΔlacI ΔspeE ΔspeG ΔargI ΔpuuP ΔpuuA PargECBH::Ptrc PspeF-potE::Ptrc PargD::Ptrc*) prepared in Example 2-3, thus preparing an XQ43 strain (W3110 *ΔlacI ΔspeE ΔspeG ΔargI ΔpuuP ΔpuuA PargECBH::Ptrc PspeF-potE::Ptrc PargD::Ptrc PspeC::Ptrc*). Then, the cells of the recombinant microorganism were cultured on an agar plate containing chloramphenicol, while cells in which double homologous recombination occurred were selected on the agar plate. Replacement with the trc promoter was confirmed by DNA sequence analysis.

### Example 3: Production of putrescine using mutant microorganisms

Each of the mutant strains *(E. coli* K12 WL3110 mutants) of Table 1, prepared in Examples 1 and 2, was cultured in a flask in minimal R medium containing 4 g/L (NH₄)₂HPO₄, 13.5 g/L KH₂PO₄, 1.7 g/L citric acid, 0.7 g/L MgSO₄·7H₂O and 0.5% (v/v) trace metal solution (Lee, S.Y. & Chang, H.N., Biotechnol. Lett., 15: 971-974, 1993). The trace metal solution contained (per liter): 5 M HCl, 10 g FeSO₄·7H₂O, 2.25 g ZnSO₄·7H₂O, 1 g CuSO₄·5H₂O, 0.5 g MnSO₄·5H₂O, 0.23 g Na₂B₄O₇·10H₂O, 2 g CaCl₂·2H₂O, and 0.1 g (NH₄)₆Mo₇O₂₄. A solution containing glucose (100 g/l) was sterilized separately and added to the sterilized medium to a final concentration of 10 g/l.

100 µL of each of the cell cultures activated in LB medium was inoculated into minimal medium, and then cultured at 30 °C at 220 rpm for 24 hours, until the maximum OD₆₀₀ reached 5. Then, 1 ml of the culture broth was added to a 350-mL baffled flask containing 50 mL of the same medium, and then was cultured at 30 °C at 220 rpm for 15 hours. The cells were separated by centrifugation, and the supernatant was analyzed by HPLC. Amines contained in the supernatant were detected by ophthaldialdehyde (OPA) derivation in a Hewlett Packard 1100 Series system (230 nm) using a C18-reverse phase column (buffer A: 45% 0.1 M sodium acetate, pH 7.2; buffer B: methanol. The analysis was carried out in the following conditions: 1-6 min 100% buffer A equilibration, 6-10 min linear gradient from 0 to 30% buffer B, 10-15 min gradient from 30% to 50% buffer B, 15-19 min gradient from 50% to 100% buffer B, 19-23 min gradient to 100% buffer B, and 23-25 min gradient from 100% to 30% buffer B, 25-28 min from 30% B to 100% A with a flow rate of 0.8 ml/min). Herein, a standard was used for calibration, and the measured concentrations of putrescine are shown in Table 1 below.

**Table 1**

| Strain | Genotype | Putrescine concentration (mg/L) |
|---|---|---|
| WL3110 | W3110 Δ*lcicI* | 0 |
| XQ08 | W3110 Δ*lacI* Δ*speE* | 0.65 |
| XQ17 | W3110 Δ*lacI* Δ*puuA* | 0 |
| XQ22 | W3110 Δ*lacI* Δ*puuP* Δ*puu*Δ | 0.6 |
| XQ23 | W3110 Δ*lacI* Δ*spaE* Δ*speG* Δ*argI* | 1.8 |
| XQ26 | W3110 Δ*lacI* Δ*speE* Δ*speG* Δ*argI* Δ*puuP* Δ*puuA* | 8.5 |
| XQ27 | W3110 Δ*lacI* Δ*speE* Δ*speG* Δ*ygjG* Δ*puuP* Δ*puuA* | 4.2 |
| XQ29 | W3110 Δ*lacI* Δ*speE* Δ*speG* Δ*ygjG* Δ*argI* Δ*puuP* Δ*puuA* | 8.5 |
| XQ33 | W3110 Δ*lacI* Δ*speE* Δ*speG* Δ*argI* Δ*puuP* Δ*puuA PargECBH::Ptrc* | 28.3 |
| XQ37 | W3110 Δ*lacI* Δ*speE* Δ*speG* Δ*argI* Δ*puuP* Δ*puuA PargECBH::Ptrc PspeF-potE::Ptrc* | 510 |
| XQ39 | W3110 *ΔlacI ΔspeE ΔspeG ΔargI* Δ*puuP* Δ*puuA* Parg*ECBH::Ptrc PspeF-potE::Ptrc PargD::Ptrc* | 820 |
| XQ43 | W3110 *ΔlacI ΔspeE ΔspeG ΔargI ΔpuuP ΔpuuA PargECBH::Ptrc PspeF-potE::Ptrc PargD::Ptrc PspeC::Ptrc* | 827 |

As can be seen in Table 1, in the mutant microorganisms in which the genes (*puuP, puuA, speE, speG,* and *argI*) involved in the putrescine degradation or utilization pathway have been deleted, the abilities of the mutant microorganisms to produce putrescine were increased depending on the kind and number of the deleted genes. Also, it could be seen that the abilities of the mutant microorganisms to produce putrescine were further increased when the promoters of the gene (*argECBH*) encoding the operone for arginine biosynthesis, the gene encoding acetylornithine aminotransferase, the gene (*speF-potE*) encoding inducible ornithine decarboxylase and putrescine/ornithine antiporter and the gene *(speC)* encoding ornithine decarboxylase were replaced with the strong promoter.

### Example 4: Amplification of gene (speC) encoding ornithine decarboxylase

### 4-1: Preparation of plasmid pKKSpeC

Ornithine decarboxylase encoding the *speC* gene of constitutive biosynthetic *E.coli* W3110 was cloned into the pKK223-3 expression vector (Pharmacia Biotech, Uppsala, Sweden) which uses the tac promoter for gene expression. PCR was performed with primers of SEQ ID NOS: 34 and 35 using the genomic DNA of *E. coli* W3110 (derived from *E. coli* K-12, λ⁻, F⁻, prototrophic) as a template, thus preparing a *speC* fragment (2156 bp).
SEQ ID NO: 34: 5'-CAGCGAATTCATGAAATCAATGAATATTGCC-3'
SEQ ID NO: 35: 5'-CATTCTGCAGTTACTTCAACACATAACCGTA-3'

Next, the prepared speC fragment (2156 bp) and the pKK223-3 plasmid were treated with restriction enzymes (EcoRI and PstI), and then with T4 DNA ligase, and the speC fragment and the pKK223-3 plasmid, digested with the restriction enzymes, were fused with each other, thus preparing a recombinant plasmid vector pKKSpeC of high copy number.

### 4-2: Preparation of plasmid p15SpeC

Ornithine decarboxylase encoding the *speC* gene of constitutive biosynthetic *E.coli* W3110 was cloned into the pTac15K expression vector (p15A origin, low copies, KmR; KAISTMBEL stock) which uses the tac promoter for gene expression. PCR was performed with primers of SEQ ID NOS: 34 and 35 using the genomic DNA of *E. coli* W3110 (derived from *E. coli* K-12, λ⁻, F⁻, prototrophic) as a template, thus preparing a *speC* fragment (2156 bp).

Next, the prepared speC fragment (2156 bp) and the pTac15K plasmid were treated with restriction enzymes (EcoRI and PstI), and then with T4 DNA ligase, and the speC fragment and the pTac15K plasmid, digested with the restriction enzymes, were fused with each other, thus preparing a recombinant plasmid vector p15SpeC of low copy number.

### 4-3: Preparation of WL3110/pKKSpeC strain

The pKKSpeC vector prepared in Example 4-1 was introduced into the WL3110 strain prepared in Example 1-1, thus a WL3110/pKKSpeC strain. Then, the prepared strain was cultured on agar solid medium containing ampicillin, while recombinant mutant microorganisms were selected.

### 4-4: Preparation of XQ 17/pKKSpeC strain

The pKKSpeC vector prepared in Example 4-1 was introduced into the XQ17 strain prepared in Example 1-3, thus an XQ17/pKKSpeC strain. Then, the prepared strain was cultured on agar solid medium containing ampicillin, while recombinant mutant microorganisms were selected.

### 4-5: Preparation of XQ22/pKKSpeC strain

The pKKSpeC vector prepared in Example 4-1 was introduced into the XQ22 straun prepared in Example 1-4, thus an XQ22/pKKSpeC strain. Then, the prepared strain was cultured on agar solid medium containing ampicillin, while recombinant mutant microorganisms were selected.

### 4-6: Preparation of XQ26/pKKSpeC strain

The pKKSpeC vector prepared in Example 4-1 was introduced into the XQ26 strain prepared in Example 1-6, thus an XQ26/pKKSpeC strain. Then, the prepared strain was cultured on agar solid medium containing ampicillin, while recombinant mutant microorganisms were selected.

### 4-7: Preparation of XQ33/pKKSpeC strain

The pKKSpeC vector prepared in Example 4-1 was introduced into the XQ33 strain prepared in Example 2-1, thus an XQ33/pKKSpeC strain. Then, the prepared strain was cultured on agar solid medium containing ampicillin, while recombinant mutant microorganisms were selected.

### 4-8: Preparation of XQ37/pKKSpeC strain

The pKKSpeC vector prepared in Example 4-1 was introduced into the XQ37 strain prepared in Example 2-2, thus an XQ37/pKKSpeC strain. Then, the prepared strain was cultured on agar solid medium containing ampicillin, while recombinant mutant microorganisms were selected.

### 4-9: Preparation of XQ39/pKKSpeC strain

The pKKSpeC vector prepared in Example 4-1 was introduced into the XQ39 strain prepared in Example 2-3, thus an XQ39/pKKSpeC strain. Then, the prepared strain was cultured on agar solid medium containing ampicillin, while recombinant mutant microorganisms were selected.

### 4-10: Preparation of XQ43/pKKSpeC strain

The p15SpeC vector prepared in Example 4-2 was introduced into the XQ43 strain prepared in Example 2-4, thus an XQ43/pKKSpeC strain. Then, the prepared strain was cultured on agar solid medium containing ampicillin, while recombinant mutant microorganisms were selected.

### Example 5: Production of putrescine using mutant microorganism in which gene (speC) encoding ornithine decarboxylase has been amplified

Each of the mutant strains prepared in Example 4 were cultured in a shake flask containing the same medium as described in Example 3. 100 µL of each of the cell cultures activated in LB medium was inoculated into minimal medium, and then cultured at 30 °C at 220 rpm for 30 hours, until the maximum OD₆₀₀ reached 5. Next, 1 ml of the culture broth was added to a 350-mL baffled flask containing 50 ml of the same medium, and then cultured at 30 °C at 220 rpm for 27 hours. The cells were separated by centrifugation, and the supernatant was analyzed by HPLC in the same conditions as described in Example 3. The analysis results are shown in Table 2.

**Table 2**

| Strain/plasmid | Putrescine concentration(mg/L) |
|---|---|
| WL3110/pKKSpeC | 220 |
| XQ17/pKKSpeC | 368 |
| XQ22/pKKSpeC | 400 |
| XQ26/pKKSpeC | 433 |
| XQ33/pKKSpeC | 910 |
| XQ37/pKKSpeC | 1100 |
| XQ39/pKKSpeC | 1189 |
| XQ43/p15SpeC | 1317 |

As can be seen in Table 2, the putrescine-producing abilities of the mutant microorganisms (WL3110/pKKSpeC, XQ17/pKKSpeC, XQ22/pKKSpeC, XQ26/pKKSpeC, XQ33/pKKSpeC, XQ37/pKKSpeC, XQ39/pKKSpeC and XQ43/p15SpeC) having reduced putrescine degradation and utilization activities were significantly increased compared to those of the mutant microorganisms (WL3110, XQ17, XQ22, XQ26, XQ33, XQ37, XQ39 and XQ43) of Table 1 in which neither pKKSpeC nor p15SpeC was introduced, when they were co-expressed with ornithine decarboxylase speC.

### Example 6: Preparation of putrescine through fed-batch culture of XQ37/pKKSpeC strain

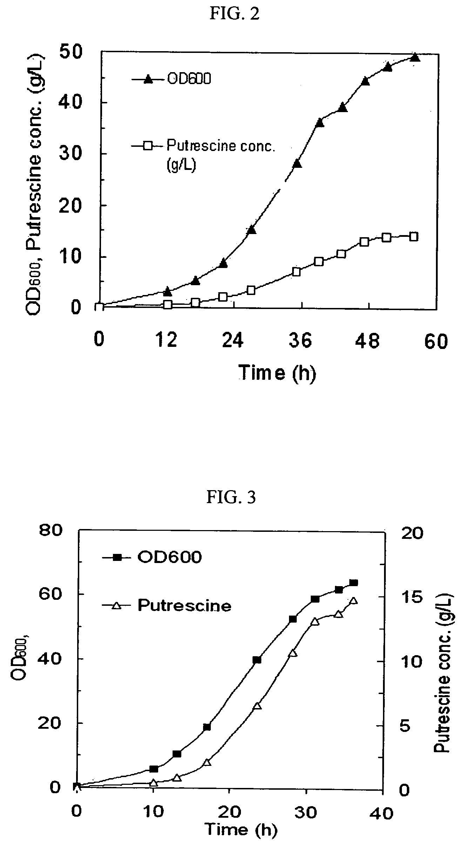

The potential of reduced putrescine degradation and utilization activity was analyzed together with decarboxylase activity in fed-batch fermentation. The fed-batch fermentation was performed in a 6.6-liter fermentor (Bioflo 3000; New Brunswick Scientific Co., Edison, NJ) after adding 10 g/l glucose to 2 liters of minimal R medium. 1 ml of the XQ37/pKKSpeC culture activated in LB medium was added to a 350-mL baffled flask containing 50 ml of the same medium, and then cultured at 30 °C at 220 rpm for 24 hours, until the maximum OD₆₀₀ reached 5. 200 ml of the preculture was subsequently used for inoculation into the fermentor. Dissolved oxygen in the fermented broth was maintained with 20% saturated air by automatically increasing an agitation speed of 850 rpm. When the pH of the fermented broth was increased by about 0.2 pH units from a fixed pH of 6.8 as a result of glucose exhaustion, the glucose-containing solution was automatically added in order to increase the glucose concentration to more than 3 g/l. The glucose-containing solution contained 500 g/l glucose and 200 g/l (NH₄)₂SO₄. Throughout the entire fermentation period except for a short time when pH was increased due to glucose exhaustion, the pH of the fermented broth was maintained at a pH of 6.8 by adding 28% (v/v) ammonia solution. The fermented broth was sampled and centrifuged to separate the cells, and the supernatant was analyzed by HPLC in the same manner as described in Example 3. The analysis results are shown in FIG. 2. As shown in FIG. 2, the XQ37/pKKSpeC strain produced 14.3 g/l of putrescine during 55.8 hours after inoculation, and the maximum putrescine productivity was 0.28 g L⁻¹ h⁻¹ after 47 hours after inoculation.

### Example 7: Production of putrescine through fed-batch culture of XQ39 strain

Fed-batch fermentation was carried out in the same manner as described in Example 6, except that the XQ39 strain was used instead of the XQ37/pKKSpeC. The fermented broth was analyzed by HPLC, and the analysis results are shown in FIG. 3. As shown in FIG. 3, the XQ39 strain produced 14.7 g/l during 36 hours after inoculation, and the maximum putrescine productivity was 0.42 g L⁻¹ h⁻¹ after 31 hours after inoculation.

### Example 8: Production of putrescine through fed-batch culture of XQ43 strain

The XQ43 strain was cultured in a flask in 50 ml of minimal R/2 (containing 2 g/L (NH₄)₂HPO₄, 6.75 g/L KH₂PO₄, 0.85 g/L citric acid, 0.7 g/L MgSO₄·7H₂O, 0.5% (v/v) trace metal solution) (Qian et al., Biotechnol. and Bioeng, 101(3): 587-601, 2008) supplemented with 3 g/L of (NH₄)₂SO₄. The trace metal solution contained (per liter): 5 M HCl, 10 g FeSO₄·7H₂O, 2.25 g ZnSO₄·7H₂O, 1 g CuSO₄·5H₂O, 0.5 g MnSO₄·5H₂O, 0.23 g Na₂B₄O₇·10H₂O, 2 g CaCl₂·2H₂O, and 0.1 g (NH₄)₆Mo₇O₂₄. A solution containing glucose (100 g/l) was sterilized separately and added to the sterilized medium to a final concentration of 10 g/l. 1 ml of the XQ43 culture activated in LB medium was added to a 350-mL baffled flask containing 50 ml of the above-described medium, and then cultured at 37 °C at 220 rpm for 24 hours, until the OD₆₀₀ of the culture reached 3.3. 200 ml of the preculture was subsequently used for inoculation into a fermentor. Dissolved oxygen in the culture was maintained with 20% saturated air by automatically increasing an agitation speed of 1000 rpm.

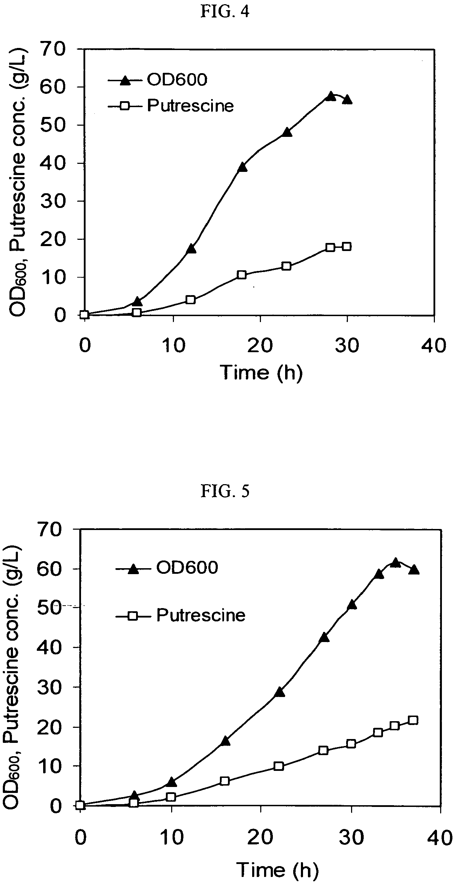

The fed-batch fermentation of the XQ43 strain was carried out in a 6.6-liter fermentor (Bioflo 3000; New Brunswick Scientific Co., Edison, NJ) after adding 10 g/l of glucose. When the pH of the fermented broth was increased by about 0.01 pH units from a fixed pH of 6.8 as a result of glucose exhaustion, a glucose-containing solution was automatically added in order to increase the glucose concentration to more than 2 g/l. The glucose-containing solution contained 522 g/l of glucose, 8 g/L of MgSO₄ and 170 g/L of (NH₄)₂SO₄. Throughout the entire fermentation period except for a short time when pH was increased due to glucose exhaustion, the pH of the fermented broth was maintained at 6.8 by adding 10 M KOH solution. The fermented broth was sampled and centrifuged to separate the cells, and the supernatant was analyzed by HPLC in the same manner as described in Example 3. The analysis results are shown in FIG. 4. As shown in FIG. 4, the XQ43 strain produced 18.0 g/l of putrescine during 30 hours after inoculation, and the maximum putrescine productivity was 0.63 g L⁻¹ h⁻¹ after 28 hours after inoculation.

### Example 9: Production of putrescine through fed-batch culture of XQ43/p15SpeC strain

Fed-batch fermentation was carried out in the same manner as described in Example 8, except that the XQ43/p15SpeC strain was used instead of the XQ43 strain. The fermented broth was analyzed by HPLC, and the analysis results are shown in FIG. 5. As shown in FIG. 5, the XQ43/p15SpeC strain produced 21.7 g/l of putrescine during 37 hours after inoculation, and the maximum putrescine productivity was 0.58g L⁻¹ h⁻¹ after 37 hours after inoculation.

### INDUSTRIAL APPLICABILITY

As described in detail above, the present invention provides mutant microorganisms having the ability to produce putrescine. These mutant microorganisms are useful for producing a high yield of putrescine which is used in a wide range of industrial applications.

## Claims

1. A mutant microorganism having the ability to produce putrescine, wherein at least one gene selected from the group consisting of a *speE* gene encoding spermidine synthase, a *speG* gene encoding spermidine N-acetyltransferase, an *argI* gene encoding ornithine carbamoyltransferase chain I-monomer and a *puuP* gene encoding putrescine importer, which are involved in the putrescine degradation or utilization pathway, is inactivated or deleted; and wherein at least one gene selected from the group consisting of a *puuA* gene encoding γ-glutamylputrescine synthase, a *ygjG* gene encoding putrescine transaminase and an *argF* gene encoding ornithine carbamoyltransferase F is further inactivated or deleted; and wherein a promoter of at least one gene selected from the group consisting of an *argECBH* gene encoding an operon for arginine biosynthesis, an *argD* gene encoding acetylornithine aminotransferase, and a *speF-potE* gene encoding inducible ornithine decarboxylase and putrescine/ornithine antiporter is replaced with a promoter selected from the group consisting of a trc promoter, a tac promoter, a T7 promoter, a lac promoter and a trp promoter; and selected from the group consisting of *Bacillus sp.* and *Escherichia sp..*

2. The mutant microorganism of claim 1, wherein a *speC* gene encoding ornithine decarboxylase is further introduced or amplified.

3. A method of preparing a mutant microorganism selected from the group consisting of *Bacillus sp.* and *Escherichia sp.* having the ability to produce putrescine, the method comprising: inactivating or deleting at least one gene selected from the group consisting of a *speE* gene encoding spermidine synthase, a *speG* gene encoding spermidine N-acetyltransferase, an *argI* gene encoding ornithine carbamoyltransferase I and a *puuP* gene encoding putrescine importer, which are involved in the putrescine degradation or utilization pathway, from a microorganism having a putrescine production pathway; and at least one gene selected from the group consisting of a *puuA* gene encoding γ-glutamylputrescine synthase, a *ygjG* gene encoding putrescine transaminase and an *argF* gene encoding ornithine carbamoyltransferase F is further inactivated or deleted.

4. A method of preparing a mutant microorganism having the ability to produce putrescine according to claim 3, further comprising introducing or amplifying a *speC* gene encoding ornithine decarboxylase, before or after the inactivation or deletion.

5. The method of preparing a mutant microorganism having the ability to produce putrescine according to claim 3, further comprising replacing a promoter of at least one gene selected from the group consisting of an *argECBH* gene encoding an operon for arginine biosynthesis, an *argD* gene encoding acetylornithine aminotransferase, and a *speF-potE* gene encoding inducible ornithine decarboxylase and putrescine/ornithine antiporter with a promoter selected from the group consisting of a trc promoter, a tac promoter, a T7 promoter, a lac promoter and a trp promoter.

6. The method of preparing a mutant microorganism having the ability to produce putrescine according to claim 5, further comprising introducing or amplifying a *speC* gene encoding ornithine decarboxylase.

7. A method for producing putrescine, the method comprising: culturing the mutant microorganism of any one of claims 1 to 2 to produce putrescine, and recovering putrescine from the culture broth.

## Patentansprüche

1. Mutierter Mikroorganismus, der die Fähigkeit hat, Putrescin zu produzieren, wobei wenigstens ein Gen, ausgewählt aus der Gruppe, bestehend aus einem *speE-Gen,* das Spermidinsynthase codiert, einem *speG-Gen,* das Spermidin-N-acetyltransferase codiert, einem *argI-Gen,* das Ornithincarbamoyltransferase-Kette-I-Monomer codiert, und einem *puuP-*Gen, das Putrescin-Importer codiert, die im Putrescin-Abbauoder -Verwertungsweg involviert sind, inaktiviert oder deletiert ist, und wobei weiterhin wenigstens ein Gen, ausgewählt aus der Gruppe, bestehend aus einem *puuA*-Gen, das γ-Glutamylputrescinsynthase codiert, einem *ygjG-Gen,* das Putrescintransaminase codiert, und einem *argF*-Gen, das Ornithincarbamoyltransferase F codiert, inaktiviert oder deletiert ist, und wobei ein Promotor von wenigstens einem Gen, ausgewählt aus der Gruppe, bestehend aus einem *argECBH*-Gen, das ein Operon für Argininbiosynthese codiert, einem *argD-*Gen, das Acetylornithinaminotransferase codiert, und einem *speF-potE-Gen,* das induzierbare Ornithindecarboxylase und Putrescin/Ornithin-Antiporter codiert, durch einen Promotor, ausgewählt aus der Gruppe, bestehend aus einem trc-Promotor, einem tac-Promotor, einem T7-Promotor, einem lac-Promotor und einem trp-Promotor, ersetzt ist, und der aus der Gruppe, bestehend aus *Bacillus sp.* und *Escherichia sp.,* ausgewählt ist.

2. Mutierter Mikroorganismus gemäß Anspruch 1, wobei ein speC-Gen, das Ornithindecarboxylase codiert, außerdem eingeführt oder amplifiziert ist.

3. Verfahren zur Herstellung eines mutierten Mikroorganismus, der aus der Gruppe, bestehend aus *Bacillus sp.* und *Escherichia sp.,* ausgewählt ist und der die Fähigkeit hat, Putrescin zu produzieren, wobei das Verfahren umfasst: Inaktivieren oder Deletieren wenigstens eines Gens, ausgewählt aus der Gruppe, bestehend aus einem *speE*-Gen, das Spermidinsynthase codiert, einem *speG-Gen,* das Spermidin-N-acetyltransferase codiert, einem *argI*-Gen, das Ornithincarbamoyltransferase-I codiert, und einem *puuP*-Gen, das Putrescin-Importer codiert, wobei diese im Putrescin-Abbau- oder -Verwertungsweg involviert sind, aus einem Mikroorganismus, der einen Putrescinproduktionsstoffwechselweg hat, und wobei außerdem wenigstens ein Gen, ausgewählt aus der Gruppe, bestehend aus einem *puuA*-Gen, das γ-Glutamylputrescinsynthase codiert, einem *ygjG*-Gen, das Putrescintransaminase codiert, und einem *argF*-Gen, das Ornithincarbarnoyltransferase F codiert, inaktiviert oder deletiert wird.

4. Verfahren zur Herstellung eines mutierten Mikroorganismus, der die Fähigkeit hat, Putrescrin zu produzieren, gemäß Anspruch 3, das außerdem Einführen oder Amplifizieren eines *speC-Gens,* das Ornithindecarboxylase codiert, vor oder nach der Inaktivierung oder Deletion umfasst.

5. Verfahren zur Herstellung eines mutierten Mikroorganismus, der die Fähigkeit, Putrescin zu produzieren hat, gemäß Anspruch 3, das außerdem Ersetzen eines Promotors wenigstens eines Gens, ausgewählt aus der Gruppe, bestehend aus einem *argECBH*-Gen, das ein Operon für Argininbiosynthese codiert, einem *argD*-Gen, das Acetylornithinaminotransferase codiert, und einem *speF-potE-Gen,* das induzierbare Ornithindecarboxylase und Putrescin/Ornithin-Antiporter codiert, durch einen Promotor, der aus der Gruppe, bestehend aus einem trc-Promotor, einem tac-Promotor, einem T7-Promotor, einem lacPromotor und einem trp-Promotor, ausgewählt wird, umfasst.

6. Verfahren zur Herstellung eines mutierten Mikroorganismus, der die Fähigkeit, Putrescin zu produzieren hat, gemäß Anspruch 5, das außerdem Einführen oder Amplifizieren eines speC-Gens, das Ornithindecarboxylase codiert, umfasst.

7. Verfahren zum Produzieren von Putrescin, wobei das Verfahren umfasst: Kultivieren des mutierten Mikroorganismus gemäß einem der Ansprüche 1 bis 2, um Putrescin zu produzieren, und Gewinnen von Putrescin aus der Kulturbrühe.

## Revendications

1. Micro-organisme mutant doté de la capacité de produire de la putrescine, dans lequel au moins un gène choisi dans le groupe constitué d'un gène *speE* codant pour la spermidine synthase, d'un gène *speG* codant pour la spermidine N-acétyltransférase, d'un gène *argI* codant pour le monomère de la chaîne I de l'ornithine carbamoyltransférase et d'un gène *puuP* codant pour l'importateur de putrescine, qui sont impliqués dans la voie de dégradation ou d'utilisation de la putrescine, est inactivé ou délété ; et dans lequel au moins un gène choisi dans le groupe constitué d'un gène *puuA* codant pour la γ-glutamylputrescine synthase, d'un gène *ygjG* codant pour la putrescine transaminase et d'un gène *argF* codant pour l'ornithine carbamoyl-transférase F est en outre inactivé ou délété ; et dans lequel un promoteur d'au moins un gène choisi dans le groupe constitué d'un gène *argECBH* codant pour un opéron pour la biosynthèse de l'arginine, d'un gène *argD* codant pour l'acétylornithine aminotransférase, et d'un gène *speF-potE* codant pour l'ornithine décarboxylase inductible et l'antiporteur de putrescine/ornithine, est remplacé par un promoteur choisi dans le groupe constitué d'un promoteur trc, d'un promoteur tac, d'un promoteur T7, d'un promoteur lac et d'un promoteur trp ; et choisi dans le groupe constitué de *Bacillus sp.* et d'*Escherichia sp*.

2. Micro-organisme mutant selon la revendication 1, dans lequel un gène *speC* codant pour l'ornithine décarboxylase est en outre introduit ou amplifié.

3. Procédé de préparation d'un micro-organisme mutant choisi dans le groupe constitué de *Bacillus sp.* et d'*Escherichia sp.* doté de la capacité de produire de la putrescine, le procédé comprenant : l'inactivation ou la délétion d'au moins un gène choisi dans le groupe constitué d'un gène *speE* codant pour la spermidine synthase, d'un gène *speG* codant pour la spermidine N-acétyltransférase, d'un gène *argI* codant pour le monomère de la chaîne I de l'ornithine carbamoyl-transférase et d'un gène *puuP* codant pour l'importateur de putrescine, qui sont impliqués dans la voie de dégradation ou d'utilisation de la putrescine, à partir d'un micro-organisme doté d'une voie de production de putrescine ; et au moins un gène choisi dans le groupe constitué d'un gène *puuA* codant pour la γ-glutamylputrescine synthase, d'un gène *ygjG* codant pour la putrescine transaminase et d'un gène *argF* codant pour l'ornithine carbamoyltransférase F est en outre inactivé ou délété.

4. Procédé de préparation d'un micro-organisme mutant doté de la capacité de produire de la putrescine selon la revendication 3, comprenant en outre l'introduction ou l'amplification d'un gène *speC* codant pour l'ornithine décarboxylase, avant ou après l'inactivation ou la délétion.

5. Procédé de préparation d'un micro-organisme mutant doté de la capacité de produire de la putrescine selon la revendication 3, comprenant en outre le remplacement d'un promoteur d'au moins un gène choisi dans le groupe constitué d'un gène *argECBH* codant pour un opéron pour la biosynthèse de l'arginine, d'un gène *argD* codant pour l'acétylornithine aminotransférase, et d'un gène *speF-potE* codant pour l'ornithine décarboxylase inductible et l'antiporteur -de putrescine/ornithine, par un promoteur choisi dans le groupe constitué d'un promoteur trc, d'un promoteur tac, d'un promoteur T7, d'un promoteur lac et d'un promoteur trp.

6. Procédé de préparation d'un micro-organisme mutant doté de la capacité de produire de la putrescine selon la revendication 5, comprenant en outre l'introduction ou l'amplification d'un gène *speC* codant pour l'ornithine décarboxylase.

7. Procédé de production de putrescine, le procédé comprenant : la culture du micro-organisme mutant selon l'une quelconque des revendications 1 à 2 pour produire de la putrescine, et la récupération de la putrescine à partir du bouillon de culture.


### SEQUENCE LISTING

<110> Korea Advanced Institute of science and Technology
<120> MUTANT MICROORGANISMS HAVING HIGH ABILITY TO PRODUCE PUTRESCINE AND METHOD FOR PRODUCING PUTRESCINE USING THE SAME
<130> 20693EP
<140> PCT/KR2009/001103
   <141> 2009-03-05
<150> 10-2008-0033125
   <151> 2008-04-10
<160> 40
<170> PatentIn version 3.2
<210> 1
   <211> 73
   <212> DNA
   <213> Artificial
<220>
   <223> primer 1
<400> 1
   gtgaaaccag taacgttata cgatrtcgca gagtatgccg gtgtctctta gattggcagc 60
   attacacgtc ttg 73
<210> 2
   <211> 70
   <212> DNA
   <213> Artificial
<220>
   <223> primer 2
<400> 2
   tcactgcccg ctttccagtc gggaaacctg tcgtgccagc tgcattaatg cacttaacgg 60
   ctgacatggg 70
<210> 3
   <211> 70
   <212> DNA
   <213> Artificial
<220>
   <223> primer 3
<400> 3
   cgcctgaata atttcggttg agagatggcg taaggcgtcg ttatctgtcg gacactatag 60
   aacgcggccg 70
<210> 4
   <211> 70
   <212> DNA
   <213> Artificial
<220>
   <223> primer 4
<400> 4
   atgttgcgcc ctttttttac gggtgttaac aaaggaggta tcaacccatg ccgcataggc 60
   cactagtgga 70
<210> 5
   <211> 70
   <212> DNA
   <213> Artificial
<220>
   <223> primer 5
<400> 5
   gatgaaacaa ccccgcaagg ggtattacgc gtttttcaac atccactcaa gacactatag 60
   aacgcggccg 70
<210> 6
   <211> 70
   <212> DNA
   <213> Artificial
<220>
   <223> primer 6
<400> 6
   cgagcggaaa acaaaccaaa ggcgaagaat catggaaacc aatatcgttg ccgcataggc 60
   cactagtgga 70
<210> 7
   <211> 70
   <212> DNA
   <213> Artificial
<220>
   <223> primer 7
<400> 7
   tcaccatcat acaacggcac tttgcgatag cggcggatca gataccataa gacactatag 60
   aacgcggccg 70
<210> 8
   <211> 70
   <212> DNA
   <213> Artificial
<220>
   <223> primer 8
<400> 8
   cgcctgaata atttcggttg agagatggcg taaggcgtcg ttatctgtcg gacactatag 60
   aacgcggccg 70
<210> 9
   <211> 70
   <212> DNA
   <213> Artificial
<220>
   <223> primer 9
<400> 9
   atgttgcgcc ctttttttac gggtgttaac aaaggaggta tcaacccatg ccgcataggc 60
   cactagtgga 70
<210> 10
   <211> 69
   <212> DNA
   <213> Artificial
<220>
   <223> primer 10
<400> 10
   aatgtaagga cacgttatgc caagcgccca cagtgttaag ctacgcccgg acactataga 60
   acgcggccg 69
<210> 11
   <211> 70
   <212> DNA
   <213> Artificial
<220>
   <223> primer 11
<400> 11
   ctattgtgcg gtcggcttca ggagagtctg acccggtgtt ttgtgctctg ccgcataggc 60
   cactagtgga 70
<210> 12
   <211> 70
   <212> DNA
   <213> Artificial
<220>
   <223> primer 12
<400> 12
   taatgtgatg ccgggatggt ttgtatttcc cggcatcttt atagcgatag gacactatag 60
   aacgcggccg 70
<210> 13
   <211> 70
   <212> DNA
   <213> Artificial
<220>
   <223> primer 13
<400> 13
   ccatataaat tgaattttaa ttcattgagg cgttagccac aggagggatc ccgcataggc 60
   cactagtgga 70
<210> 14
   <211> 71
   <212> DNA
   <213> Artificial
<220>
   <223> primer 14
<400> 14
   atagcaatag aacactttgg gtggaagaat agacctatca ctgcataaaa taatgtgatg 60
   ccgggatggt t 71
<210> 15
   <211> 70
   <212> DNA
   <213> Artificial
<220>
   <223> primer 15
<400> 15
   ccacctttgt gacaaagatt tatgctttag acttgcaaat gaataatcat ccatataaat 60
   tgaattttaa 70
<210> 16
   <211> 70
   <212> DNA
   <213> Artificial
<220>
   <223> primer 16
<400> 16
   ctgcaatact taaatcggta tcatgtgata cgcgagcctc cggagcatat gacactatag 60
   aacgcggccg 70
<210> 17
   <211> 70
   <212> DNA
   <213> Artificial
<220>
   <223> primer 17
<400> 17
   cgtcgtatcg ccatccgatt tgatattacg cttcttcgac acttactcgc ccgcataggc 60
   cactagtgga 70
<210> 18
   <211> 75
   <212> DNA
   <213> Artificial
<220>
   <223> primer 18
<400> 18
   tatccgctca caattccaca cattatacga gccggatgat taattgtcaa cagctgacac 60
   tatagaacgc ggccg 75
<210> 19
   <211> 75
   <212> DNA
   <213> Artificial
<220>
   <223> primer 19
<400> 19
   tatccgctca caattccaca cattatacga gccggatgat taattgtcaa cagctccgca 60
   taggccacta gtgga 75
<210> 20
   <211> 75
   <212> DNA
   <213> Artificial
<220>
   <223> primer 20
<400> 20
   cgctggcacc cacaatcagc gtattcaaca tggtctgttt cctgtgtgaa attgttatcc 60
   gctcacaatt ccaca 75
<210> 21
   <211> 75
   <212> DNA
   <213> Artificial
<220>
   <223> primer 21
<400> 21
   tctcgataaa tggcggtaat ttgtttttca tggtctgttt cctgtgtgaa attgttatcc 60
   gctcacaatt ccaca 75
<210> 22
   <211> 75
   <212> DNA
   <213> Artificial
<220>
   <223> primer 22
<400> 22
   atgttcatat gcggatggcg atttacatag gtcactagct ctgcgccagc gtagccgctg 60
   gcacccacaa tcagc 75
<210> 23
   <211> 75
   <212> DNA
   <213> Artificial
<220>
   <223> primer 23
<400> 23
   tcgagtgcct cttccgtggc gcttattgaa ggtgtggcaa tcagagcgcg gtaaatctcg 60
   ataaatggcg gtaat 75
<210> 24
   <211> 62
   <212> DNA
   <213> Artificial
<220>
   <223> primer 24
<400> 24
   actaagggca cttcagcgta caggtcttcc tgactctctg tagacactat agaacgcggc 60
   cg 62
<210> 25
   <211> 60
   <212> DNA
   <213> Artificial
<220>
   <223> primer 25
<400> 25
   agcttcgact ttcacttctt caatgcccgt tagtctaccg actaagggca cttcagcgta 60
<210> 26
   <211> 75
   <212> DNA
   <213> Artificial
<220>
   <223> primer 26
<400> 26
   aatcactaac cgcaattttt aattttgaca tggtctgttt cctgtgtgaa attgttatcc 60
   gctcacaatt ccaca 75
<210> 27
   <211> 60
   <212> DNA
   <213> Artificial
<220>
   <223> primer 27
<400> 27
   aaggcggaca actcatatta tgaagtttgc tcatcgcaat agcttcgact ttcacttctt 60
<210> 28
   <211> 77
   <212> DNA
   <213> Artificial
<220>
   <223> primer 28
<400> 28
   cgactttcat taatgtagat acattctcgc tgcgtggtaa aacagtccgg gcaagaatca 60
   ctaaccgcaa tttttaa 77
<210> 29
   <211> 60
   <212> DNA
   <213> Artificial
<220>
   <223> primer 29
<400> 29
   caactgctgg ctaatttcct gcatcgctga tttctgattg gacactatag aacgcggccg 60
<210> 30
   <211> 60
   <212> DNA
   <213> Artificial
<220>
   <223> primer 30
<400> 30
   gcagttccat ccagaaagta ttcttagcga acaaggacat caactgctgg ctaatttcct 60
<210> 31
   <211> 75
   <212> DNA
   <213> Artificial
<220>
   <223> primer 31
<400> 31
   cgcgtgtaat tgctgtttgt tcaattgcca tggtctgttt cctgtgtgaa attgttatcc 60
   gctcacaatt ccaca 75
<210> 32
   <211> 60
   <212> DNA
   <213> Artificial
<220>
   <223> primer 32
<400> 32
   ttatggggat tcgccatcgc cagtgggatc tggaaggtgt gcagttccat ccagaaagta 60
<210> 33
   <211> 58
   <212> DNA
   <213> Artificial
<220>
   <223> primer 33
<400> 33
   cggagcataa atcggcagga tcacttcatc gaaagtcgcg cgtgtaattg ctgtttgt 58
<210> 34
   <211> 31
   <212> DNA
   <213> Artificial
<220>
   <223> primer 34
<400> 34
   cagcgaattc atgaaatcaa tgaatattgc c 31
<210> 35
   <211> 31
   <212> DNA
   <213> Artificial
<220>
   <223> primer 35
<400> 35
   cattctgcag ttacttcaac acataaccgt a 31
<210> 36
   <211> 70
   <212> DNA
   <213> Artificial
<220>
   <223> primer 36
<400> 36
   tttgcccgat gcacgccatc tccttacatt ctctcgctta tcgccgtttc gacactatag 60
   aacgcggccg 70
<210> 37
   <211> 57
   <212> DNA
   <213> Artificial
<220>
   <223> primer 37
<400> 37
   tgccatgatt gcgcgaattt tctcctctct gtacggagtt tgcccgatgc acgccat 57
<210> 38
   <211> 75
   <212> DNA
   <213> Artificial
<220>
   <223> primer 38
<400> 38
   tactggcggc aatattcatt gatttcatgg tctgtttcct gtgtgaaatt gttatccgct    60
   cacaattcca cacat                                                     75
<210> 39
   <211> 69
   <212> DNA
   <213> Artificial
<220>
   <223> primer 39
<400> 39
   gatggcttgt ttgttcgcaa agtcctggct tgcacgcttt agcgaaaggt gccatgattg    60
   cgcgaattt                                                            69
<210> 40
   <211> 75
   <212> DNA
   <213> Artificial
<220>
   <223> primer 40
<400> 40
   atctcccaac gccaccacgc gacgatgaga agaaagtcgg gataccagtt cactactggc    60
   ggcaatattc attga                                                     75